# EUROPÄISCHE PATENTANMELDUNG

(11) **EP 1 382 326 A1**
(43) Veröffentlichungstag der Anmeldung: **21.01.2004**
(21) Anmeldenummer: 02291799.1
(22) Anmeldetag: 17.07.2002
(51) Int. Cl.: A61K 7/40

(54) **Verfahren zum Schutz der Haut gegen die Alterung**

(71) Anmelder: Cognis France S.A., 31360 Saint-Martory (FR)
(72) Erfinder: Pauly, Gilles, 54000 Nancy (FR); Freis, Olga, 54280 Seichamps (FR); Danoux, Louis, 54420 Saulxures-les-Nancy (FR)
(74) Vertreter: Herrburger, Pierre

(57) **Zusammenfassung**

Vorgeschlagen wird ein Verfahren zur Herstellung von Mitteln zur Stimulation von menschlichen Hautzellen sowie zum Schutz gegen Hautalterung und schädigende Einflüsse durch Umweltgifte und UV-Strahlung, dadurch gekennzeichnet, dass die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

Des Weiteren wird die Verwendung von kosmetischen oder pharmazeutischen Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zur Stimulation menschlicher Hautzellen sowie zum Schutz gegen Hautalterung und schädigende Einflüsse durch Umweltgifte und UV-Strahlung vorgeschlagen.

Mittel und/oder Substanzen, die eine Aktivität aufweisen, die Synthese von Energiedonatoren der mitochondrialen Atmungskette zu erhöhen und trotzdem die Rate reaktiver Sauerstoffspecies (ROS) im Zellmetabolismus zu erniedrigen können beispielsweise Pflanzenextrakte sein oder auch Mischungen von unterschiedlichen Aktivstoffen.

Bevorzugt enthalten diese Mischungen ein Antioxidans in Kombination mit mindestens einem weiteren Wirkstoff, besonders bevorzugt enthalten sie Vitamin C in Kombination mit Hefe und Glycogen. Es wurde gefunden, dass sich durch die Kombination von Vitamin C, Hefe und Glycogen synergistische Effekte ergeben.

## Beschreibung

### Gebiet der Erfindung

Die Erfindung befindet sich auf dem Gebiet der kosmetischen und pharmazeutischen Zubereitungen und betrifft ein Verfahren zum Schutz der menschlichen Haut gegen die Alterung und gegen schädigende Einflüsse durch Umweltgifte und UV-Strahlung. Des weiteren betrifft die Erfindung die Verwendung von kosmetischen oder pharmazeutischen Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zur Stimulation menschlicher Hautzellen und zum Schutz gegen Hautalterung, toxische Umwelteinflüsse und UV-Strahlung.

### Stand der Technik

Mitochondrien sind intrazelluläre Organellen, die die wichtigsten Elemente für die Energiegewinnung der Zelle darstellen. Sie enthalten eine vollständige Enzymausstattung für den Fettsäureabbau, den Citronensäurezyklus, die oxidative Phosphorylierung und die Atmungskette und sind hauptsächlich verantwortlich für Energielieferung und die Oxidation unterschiedlicher Moleküle im Endstadium des aeroben Metabolismus von Zellen.

Damit verbunden sind diese Organellen ein sehr empfindlicher Anzeiger für Stressfaktoren wie toxische Umweltgifte, UV-A oder UV-B-Lichteinfluß und auch natürliche Alterung. Stress vermindert die Kapazität der Mitochondrien Energie zu produzieren. So geschädigte Mitochondrien setzen eine erhöhte Menge sogenannter reaktiver Sauerstoffspecies (ROS - reactive oxygen species), sowie weitere Faktoren wie Cytochrom C, das den Zelltod in Form der Apoptose auslöst, frei. Diese ROS werden in der mitochondrialen Atmungskette grundsätzlich als Nebenprodukte des Elektronentransportes freigesetzt. So entsteht beispielweise bei der Umsetzung von Succinat, dem effektivsten Substrat der Atmungskette, Wasserstoffperoxid. Die ROS werden wiederum durch einen Schutzmechanismus der Zellen in Form von Enzymen, die freie Sauerstoffradikale binden resp. umsetzen, abgefangen, da sie ansonsten zelluläre Makromoleküle wie Proteine, Lipide und Nukleinsäuren schädigen würden.

Die Rate an ROS in den Zellen ist jedoch bei zunehmender Alterung erhöht, da die Aktivität der sauerstoffradikalbindenden Enzyme abnimmt und die Effektivität des Elektronentransportes in der mitochondrialen Atmungskette abnimmt.

Es ist bekannt, dass UV-A und UV-B-Strahlung durch Schädigung der Mitochondrien ebenfalls eine Erhöhung der ROS bewirken, die dann zu Schäden zellulärer Makromoleküle beiträgt.

In der Internationalen Patentanmeldung WO 98/51291 wird daher die Verwendung von Antioxidantien zum Schutz von Zellen und Gewebe vor ROS, die durch ischemische Prozesse von Mitochondrien vermehrt freigesetzt wurden, offenbart. Auch die Verwendung von L-Ergothionin zum Schutz von Mitochondrien vor oxidativen Schäden durch den Einfluss von UV-Strahlung und Umweltgiften wurde bereits beschrieben (WO 98/36748 und US 6 103 746).

Ein anderer Ansatzpunkt, den Vorgang der Alterung zu bremsen, besteht darin, die Aktivität der Mitochondrien zu erhöhen. So wurde in der Japanischen Patentanmeldung JP 08333270 gezeigt, dass die Verwendung von Extrakten der Taprafrucht die Effektivität der Mitochondrien steigert. Trotzdem wird dabei wiederum die Freisetzung von ROS gesteigert.

Es besteht daher immer noch Bedarf, Mechanismen zu finden, die eine Zellalterung oder Schädigung durch Umweltgifte oder UV-Strahlung vermindern, welches somit die Aufgabe der vorliegenden Erfindung war.

### Beschreibung der Erfindung

Gegenstand der Erfindung sind Verfahren zur Herstellung von Mitteln zur Stimulation von menschlichen Hautzellen, Mitteln zum Schutz der menschlichen Haut gegen die Alterung, Mitteln gegen schädigende Einflüsse und Hautalterung durch UV-Lichteinwirkung, sowie Mitteln zum Schutz der menschlichen Haut gegen toxische Umwelteinflüsse, dadurch gekennzeichnet, dass die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

Überraschenderweise wurde gefunden, dass durch ein Verfahren, bei dem einerseits die mitochondiale Funktion stimuliert wird und andererseits trotzdem die Rate der dabei freigesetzten reaktiven Sauerstoffspecies (ROS) nicht erhöht wird, die menschliche Haut vor der Alterung sowie vor Schäden durch Umweltgifte und UV-Strahlung effektiver geschützt werden kann. Durch die Erhöhung der Synthese von Energiedonatoren der mitochondrialen Atmungskette wie beispielsweise Adenosintriphosphat (ATP) und/oder Creatinphoshat können zellphysiologische Mechanismen unterstützt werden, die einerseits vorbeugend die Haut vor Schädigungen schützen, andererseits den Reparaturmechanismus für bereits geschädigte Haut fördern.

Während des natürlichen Alterungsprozesses nimmt die durch die Mitochondrien zur Verfügung gestellte Energie zunehmend ab und die Rate reaktiver Sauerstoffspecies, die in der Zelle nicht entgiftet werden, steigt stetig. Ebenso ist bekannt, dass durch den Einfluß von UV-Strahlung Schädigungen in der Atmungskette hervorgerufen werden, die ebenfalls zu einem verminderten Potential an Energiegewinnung und gleichzeitig einer erhöhten Rate an ROS führen.

Es war daher ein Ziel die Kapazität der Energiegewinnung gerade alternder Haut zu erhöhen. Im Zellmetabolismus ist zu beobachten, dass durch die Induktion der mitochondrialen Funktionen, wiederum die Rate der freigesetzten ROS erhöht wird. In dem Verfahren der vorliegenden Erfindung soll jedoch trotz der erhöhten Synthese von Energiedonatoren der mitochondrialen Atmungskette, die Menge an ROS aus der Atmungskette erniedrigt werden.

Ein weiterer Gegenstand der Erfindung ist die Verwendung von kosmetischen oder pharmazeutischen Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zur Stimulation menschlicher Hautzellen und/oder zum Schutz der menschlichen Haut gegen die Alterung und/oder zum Schutz der Haut gegen schädigende Einflüsse und Hautalterung durch UV-Lichteinwirkung und/oder zum Schutz der menschlichen Haut gegen toxische Umwelteinflüsse.

Es handelt es sich bei den möglichen Energiedonatoren der mitochondrialen Atmungskette um Verbindungen, die durch ihre besondere Struktur die Übertragung von chemisch gebundener Energie zwischen energieliefernden und energieverbrauchenden Prozessen übernehmen Diese sind beispielsweise Glucose-6-phopshat, Pyrophosphat, Phosphoenolpyruvat, vorzugsweise Creatinphosphat und besonders bevorzugt Adenosintriphosphat (ATP).

Mittel und/oder Substanzen, die eine Aktivität aufweisen, die Synthese von Energiedonatoren der mitochondrialen Atmungskette zu erhöhen und trotzdem die Rate reaktiver Sauerstoffspecies (ROS) im Zellmetabolismus zu erniedrigen können beispielsweise Pflanzenextrakte sein oder auch Mischungen von unterschiedlichen Aktivstoffen.

Bevorzugt enthalten diese Mischungen ein Antioxidans in Kombination mit mindestens einem weiteren Wirkstoff, besonders bevorzugt enthalten sie Vitamin C in Kombination mit Hefe und Glycogen. Es wurde gefunden, dass sich durch die Kombination von Vitamin C, Hefe und Glycogen synergistische Effekte ergeben.

Die Aktivsubstanzen sind in den Mischungen Antioxidans : Hefe : Glycogen im Verhältnis (1 - 10) : (10 - 80) : (10 - 80), vorzugsweise (3 - 8) : (20 - 70) : (20 - 60), und besonders bevorzugt (4 - 6): (40 - 60): (30 - 50) enthalten.

### Antioxidantien

Antioxidantien, die in den Mischungen zur Stimulation der Synthese von Energiedonatoren und zur Verminderung der ROS-Rate eingesetzt werden können sind beispielsweise Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe. Die darunter besonders bevorzugten Antioxidantien sind Vitamin C und seine Derivate.

### Beispiele

### Methode:

Humane Fibroblasten werden im Standardmedium mit foetalem Kälberserum (FCS-foetal calf serum) über 3 Tage kultiviert. Danach wird das Medium gegen Standardmedium ohne FCS, aber mit den zu testenden Aktivsubstanzen ausgewechselt. Nach einer Inkubation über 3 Tage wird die Zellaktivität durch Bestimmung der folgenden Parameter gemessen:
- Adenosintriphoshat (ATP) - gemessen durch Lumineszenz basierend auf dem enzymatischen Komplex Luciferin/Luciferase [Vasseur P, Aerts C., Journal Français Hydrologie 1981, 9, 149-156]
- ROS-Freisetzung aus Mitochondrien - gemessen durch Fluoreszenz mittels Dihydro-rhodamin 123 (Rh123) über die Detektion von H2O2 im zellulären Cytoplasma [H Sakurada H, Koizumi H, Ohkawara A, Ueda T Kamo N., Arch. Dermatol. Res., 1992, 284, 114-116].

Alle Werte sind normalisiert auf den zellulären Proteingehalt bestimmt durch die Methode nach Bradford [Bradford MM., Anal. Biochem. 1976, 72 ; 248-254].

### Ergebnisse:

**Tabelle 1:**

| Rate an gewonnenem ATP und freigesetzten ROS in mitochondrialer Atmungskette nach Inkubation mit unterschiedlichen Zusammensetzungen an Aktivstoffen | | |
|---|---|---|
| Eingesetzte Substanz | Rate an ATP/Protein | Rate an ROS/Protein |
| Kontrolle | 100% | 100% |
| A - 0,3 Gew.% [ Glycogen 50 Gew. % + Hefeextrakt 50 Gew. %] | 111 % | 199 % |
| B - 0,3 Gew.% [ Glycogen 45 Gew. % + Hefeextrakt 50 Gew. % + Vitamin C 5 Gew.%] | 112 % | 68 % |
| C - 0,3 Gew.% [ Glycogen 40 Gew. % + Hefeextrakt 60 Gew. %] | 117 % | 212% |
| D - 0,3 Gew.% [ Glycogen 35 Gew. % + Hefeextrakt 60 Gew. % + Vitamin C 5 Gew.%] | 121 % | 84 % |

Die Ergebnisse zeigen, dass alle Mischungen eine erhöhte Rate an ATP aufweisen, jedoch nur Mischung B und D mit Antioxidans zeigen ebenfalls eine verminderte Rate an ROS.

Einen synergistischen Effekt kann man deutlich erkennen, da der Zusatz an 5 Gew. % Vitamin C selbst die starke Erhöhung an ROS auf 212 %, die durch die erhöhte Aktivität der Mitochondrien (Erhöhung an Energiedonator ATP) bewirkt wird, nicht nur kompensiert, sondern sogar die Menge an ROS gegenüber dem Normwert vermindert ohne dabei den positiven Effekt auf die Konzentration an ATP zu verringern.

Die Ergebnisse zeigen deutlich, dass es durch die geeignete Auswahl an Aktivstoffen möglich ist, die Synthese von Energiedonatoren der mitochondrialen Atmungskette zu erhöhen und dabei trotzdem gleichzeitig die Rate reaktiver Sauerstoffspecies (ROS) im Zellmetabolismus zu erniedrigen.

Im Zellmetabolismus werden so wertvolle Energieträger zur Verfügung gestellt, die in Kombination mit der Reduzierung zellschädigender ROS zu einer Vorbeugung und Behandlung von Hautalterung und Schäden der Haut durch toxische Umwelteinflüsse oder UV-Lichteinwirkung beitragen.

## Patentansprüche

1. Verfahren zur Herstellung von Mitteln zur Stimulation von menschlichen Hautzellen, **dadurch gekennzeichnet, dass** die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

2. Verfahren zur Herstellung von Mitteln zum Schutz der menschlichen Haut gegen die Alterung, **dadurch gekennzeichnet, dass** die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

3. Verfahren zur Herstellung von Mitteln zum Schutz der menschlichen Haut gegen schädigende Einflüsse und Hautalterung durch UV-Lichteinwirkung, **dadurch gekennzeichnet, dass** die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

4. Verfahren zur Herstellung von Mitteln zum Schutz der menschlichen Haut gegen toxische Umwelteinflüsse, **dadurch gekennzeichnet, dass** die Mittel mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt.

5. Verwendung kosmetischer oder pharmazeutischer Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zur Stimulation menschlicher Hautzellen.

6. Verwendung kosmetischer oder pharmazeutischer Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zum Schutz der menschlichen Haut gegen die Alterung.

7. Verwendung kosmetischer oder pharmazeutischer Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zum Schutz der menschlichen Haut gegen schädigende Einflüsse und Hautalterung durch UV-Lichteinwirkung.

8. Verwendung kosmetischer oder pharmazeutischer Zubereitungen, die mindestens eine Substanz enthalten, welche die Synthese von Energiedonatoren der mitochondrialen Atmungskette erhöht und dabei gleichzeitig die Rate reaktiver Sauerstoffspecies (ROS) im Zellmetabolismus erniedrigt, zum Schutz der menschlichen Haut gegen toxische Umwelteinflüsse.

9. Verwendung nach mindestens einem der Ansprüche 5 bis 8, **dadurch gekennzeichnet, dass** als Energiedonatoren der mitochondrialen Atmungskette Adenosintriphosphat (ATP) und/oder Creatinphosphat erhöht werden.

10. Verwendung nach mindestens einem der Ansprüche 5 bis 9, **dadurch gekennzeichnet, dass** die Substanz ein Pflanzenextrakt ist.

11. Verwendung nach mindestens einem der Ansprüche 5 bis 10, **dadurch gekennzeichnet, dass** die Substanz ein Antioxidans in Kombination mit mindestens einem weiteren Wirkstoff ist.

12. Verwendung nach mindestens einem der Ansprüche 5 bis 11, **dadurch gekennzeichnet, dass** die Substanz Vitamin C in Kombination mit Hefe und Glycogen enthält.
